# EUROPÄISCHE PATENTSCHRIFT

(11) **EP 4 188 284 B1**
(45) Veröffentlichungstag und Bekanntmachung des Hinweises auf die Patenterteilung: **05.11.2025**
(21) Anmeldenummer: 21773634.7
(22) Anmeldetag: 15.09.2021
(51) Int. Cl.: B29C 43/18, B29C 43/52, A61F 2/30, B29C 43/00

(54) **IMPLANTIERBARE KIEFERGELENKSPROTHESE UND ENTSPRECHENDES HERSTELLUNGSVERFAHREN**
IMPLANTABLE TEMPOROMANDIBULAR JOINT PROSTHESIS AND CORRESPONDING MANUFACTURING METHOD
PROTHÈSE IMPLANTABLE DE L'ARTICULATION TEMPOROMANDIBULAIRE ET MÉTHODE DE FABRICATION CORRESPONDANTE

(30) Priorität: 16.09.2020 DE 102020211582
(43) Veröffentlichungstag der Anmeldung: 07.06.2023
(73) Patentinhaber: Karl Leibinger Asset Management GmbH & Co. KG, 78570 Mühlheim an der Donau (DE)
(72) Erfinder: AKSU, Adem, 78054 VS-Schwenningen (DE); LEIBINGER, Ralf, 78570 Mühlheim a. d. Donau (DE); REINAUER, Frank, 78576 Emmingen-Liptingen (DE); WOLFRAM, Tobias, 63303 Dreieich (DE)
(74) Vertreter: Isarpatent
(86) Internationale Anmeldenummer: PCT/EP2021/075368
(87) Internationale Veröffentlichungsnummer: WO 2022/058374

(56) Entgegenhaltungen:
- EP-A1- 0 203 719
- WO-A2-2020/141774
- CN-U- 205 626 192
- KR-B1- 102 009 324
- US-A- 6 132 466
- US-A1- 2005 146 070
- US-A1- 2020 008 945
- US-B2- 7 879 275

## Beschreibung

Die vorliegende Erfindung betrifft eine implantierbare Kiefergelenksprothese gemäß dem Oberbegriff des Anspruchs 1, wie aus der KR 102 009 324 B1 bekannt.

Derartige Kiefergelenkprothesen sind ebenfalls aus der US 6 132 466 A, der US 2020/008945 A1 und der WO 2020/141774 A2 bekannt.

Außerdem betrifft die Erfindung ein entsprechendes Verfahren zur Herstellung einer implantierbaren Kiefergelenksprothese gemäß dem Oberbegriff des Anspruchs 12, wie aus der CN 205 626 192 U bekannt.

Das Kiefergelenk (im Folgenden auch als TMJ = temporomandibular joint bezeichnet) ist eines der meist genutzten und wichtigsten Gelenke im menschlichen Körper. Es spielt eine wesentliche Rolle in der Bewegungsführung der Mandibula (des Unterkiefers), beim Kauen, Sprechen, Schlucken und der Stressbewältigung. Dabei ist es sogar im Schlaf durch die Schluckbewegung ständig in Bewegung. Damit das Kiefergelenk seine Bewegungsabläufe durchführen kann, müssen die Form der Gelenkflächen, der Zustand des Gebisses, Zahnstellung, Zahnform, die Kaumuskulatur ein funktionales System bilden, welches durch die Vielzahl an Komponenten einem gewissen Maß an Störungsanfälligkeit unterliegt. Das linke und rechte Kiefergelenk arbeiten dabei stets zusammen und bilden insoweit eine funktionelle Einheit.

Treten im Bereich des Kiefergelenks jedoch Störungen, wie z.B. Funktionseinschränkungen oder Erkrankungen, auf, kann es zu namhaften Beeinträchtigungen der Lebensqualität im Alltag von Patienten kommen. Eine Wiederherstellung von korrekten Gelenkstrukturen ist hierbei meist nur im Wege einer chirurgischen Lösung, also durch Resektion des gestörten Gelenks und dessen Ersatz durch eine Kiefergelenksprothese, möglich.

Eine Kiefergelenksprothese ist beispielsweise aus der EP 3 003 225 B1 bekannt, welche einen Mechanismus bereitstellt, der eine kombinierte Translations- und Rotationsbewegung zwischen Schädel und Unterkiefer gestattet, bei der eine gleitende Bewegung zwischen Oberflächen stattfindet. Allerdings kann dabei ein Verschleiß auftreten.

Es die Aufgabe der vorliegenden Erfindung, eine verbesserte implantierbare Kiefergelenksprothese zu schaffen, mittels der die gleitende Bewegung der Oberflächen möglichst zuverlässig und sicher, und dabei verschleißarm ausgeführt wird.

Die Aufgabe wird durch eine implantierbare Kiefergelenksprothese mit den Merkmalen des Anspruchs 1 gelöst. Die erfindungsgemäße Lösung besteht also insbesondere darin, dass bei einer Kiefergelenksprothese der eingangs genannten Art das zweite Implantatteil eine aus einem metallischen Werkstoff gebildete erste Komponente aufweist, welche an dem Cranium befestigbar ist, und eine aus einem Kunststoff gebildete zweite Komponente aufweist, welche die Gelenkfläche ausbildet, und dass die erste Komponente eine erste Verbindungsfläche aufweist und die zweite Komponente eine zweite Verbindungsfläche aufweist, wobei die erste Verbindungsfläche und die zweite Verbindungsfläche ineinandergreifend miteinander einteilig in einem Schmelzverbund verbunden sind.

Mit der erfindungsgemäßen implantierbaren Kiefergelenksprothese wird also an derjenigen Gelenkfläche, die das Widerlager für den Gelenkkopf bildet, eine zuverlässige Gleitpaarung dadurch bereitgestellt, dass an dem zweiten Implantatteil mehrere Komponenten vorgesehen sind, die mit ihren Verbindungsflächen ineinandergreifen und einteilig verbunden sind, so dass durch die Verbindung von Metallkomponente und Kunststoffkomponenten eine stabile verschleißarme Gelenkfläche für die Gleitpaarung entsteht. Insbesondere ist kein "Snap-In" oder Formschlussmechanismus vorgesehen, sondern das Widerlager als offenschalige Gleitfläche basierend auf der realen Oberfläche des menschlichen Kiefergelenks ausgeführt. Die native Muskelschlinge hält die beiden Implantatteile in Position und ermöglicht die Wiederherstellung einer realitätsnahen Gelenkfunktion.

Kondylus und Os temporale lassen sich in ihrer 3D-Kontour hinterlegen, und somit ist die erfindungsgemäße implantierbare Kiefergelenksprothese patientenspezifisch skalierbar und pathologisch anwendbar.

Bevorzugte Weiterbildungen der erfindungsgemäßen implantierbaren Kiefergelenksprothese finden sich in den entsprechenden Unteransprüchen.

Um zwischen den beiden Verbindungsflächen der ersten Komponente einen haltbaren und hinsichtlich des Quergleitens belastbaren Formschluss zu erreichen, kann bei einer bevorzugten Weiterbildung der Kiefergelenksprothese die erste Verbindungsfläche mit Ausnehmungen versehen sein, in welche an der zweiten Verbindungsfläche angeordnete Vorsprünge eingreifen.

**In** einer weiteren bevorzugten Weiterbildung können die Ausnehmungen in der ersten Verbindungsfläche der ersten Komponente und die entsprechenden Vorsprünge der zweiten Komponente zur Gewährleistung des Formschlusses der Verbindungsflächen homogen oder inhomogen ausgebildet sein. Hierbei können die Ausnehmungen sowohl hinsichtlich ihrer Formgebung als auch ihrer Verteilung an der Verbindungfläche homogen bzw. inhomogen sein. Beispielsweise kann eine homogene Variante darin bestehen, dass im Querschnitt quadratische Ausnehmungen in beide Erstreckungsrichtungen der Verbindungsfläche gleichmäßig angeordnet sind, während eine inhomogene Variante mit einer Mehrzahl von Ausnehmungsarten ausgebildet sein kann, die sich an der Verbindungsfläche etwa in unterschiedlichen Richtungen mit sich unterscheidenden Anzahlen erstrecken oder anderweitig die Gleichmäßigkeit durchbrechen. Dies gilt umgekehrt auch für die vorstehend erwähnten Vorsprünge als zu den Ausnehmungen komplementäre Elemente der zweiten Verbindungsfläche.

Bei einer weiteren bevorzugten Weiterbildung der erfindungsgemäßen Kiefergelenksprothese kann die Größe der Ausnehmungen an der ersten Verbindungsfläche der ersten Komponente beispielsweise zwischen 1 µm und 2000 µm betragen, so dass hinsichtlich der Einzelausdehnung der jeweiligen Ausnehmungen ein weiter Größenbereich abgedeckt wird. Es sind jedoch auch andere Größen von Einzelausdehnungen vorstellbar.

Bei einer weiteren bevorzugten Weiterbildung der erfindungsgemäßen Kiefergelenksprothese, bei der die Gleitpaarung des Gelenks einen möglichst geringen Platzbedarf aufweist, können die erste und zweite Verbindungsfläche als zueinander kongruente Flächen ausgebildet sein, die einander im Wesentlichen überdecken. Hierdurch kann die betreffende Gelenkfläche in genau der benötigten Ausdehnung bereitgestellt werden.

Zur Gewährleistung eines natürlichen Bewegungsablaufs mit der erfindungsgemäßen Kiefergelenksprothese kann bei einer anderen bevorzugten Weiterbildung die erste Verbindungsfläche konkav ausgebildet sein. Es sind aber auch mehreren unterschiedlichen Krümmungen mit sich unterscheidenden Krümmungsradien und verschiedene, insbesondere zueinander orthogonale, Richtungen, denkbar.

Erfindungsgemäß werden die beiden Komponenten des zweiten Implantatteils besonders stabil dadurch verbunden, dass die erste Komponente und die zweite Komponente mit ihren ineinandergreifenden Verbindungsflächen einen Schmelzverbund bilden. Neben dem durch die Gestalt der Verbindungsflächen erreichten Formschluss beruht die Stabilität dann insbesondere auf dem Stoffschluss, der z.B. durch eine Beaufschlagung der Verbindungsflächen mit Temperatur, ggf. zusätzlich unter Druck und/oder Vakuum realisiert werden kann.

Eine Gelenkfläche mit sehr guten Gleiteigenschaften wird bei einer weiteren bevorzugten Weiterbildung erreicht, dass die erste Komponente aus Titan oder einer Titanlegierung ausgebildet ist und dass der Kunststoff der zweiten Komponente aus einem thermoplastischen Kunststoff, insbesondere aus PE-UHMW als thermoplastischem Kunststoff, ausgebildet ist. Es sind aber auch andere Kombinationen von metallischen und kunststoffartigen Werkstoffen denkbar.

In einer anderen bevorzugten Weiterbildung, bei der die Gelenkfläche zusammen mit dem Gelenkkopf eine besonders gut handhabbare Gleitpaarung bildet, kann das erste Implantatteil aus einer Keramik, einem PEEK-Werkstoff, einem metallischen Werkstoff oder einer Kombination dieser Werkstoffe ausgebildet sein.

Um ein seitliches Verrutschen in einem normalen Bewegungsablauf unterbinden zu können, kann bei einer weiteren bevorzugten Weiterbildung die erste Komponente einen Randbereich aufweisen, der die zweite Komponente zumindest bereichsweise umgreift. Der Randbereich bietet eine weitere Stabilisierung der Verbindungsflächen zueinander. Der Randbereich kann dabei den Seitenenden der Gelenkfläche angeordnet sein, während deren vorderes und hinteres Stirnende von dem Randbereich frei sein können. Es sind aber auch andere Konfigurationen des Randbereichs vorstellbar.

Um die beiden Implantatteile der erfindungsgemäßen Kiefergelenksprothese in geeigneter Weise zueinander ausrichten und gleichzeitig stabil an den einander gegenüberliegenden Körperbereichen lagern zu können, können bei einer weiteren bevorzugten Weiterbildung das erste und das zweite Implantatteil mittels eines ersten bzw. zweiten Befestigungsteils an dem Unterkiefer bzw. dem Cranium lösbar befestigbar sein, wodurch auch eine spätere Entfernung bzw. ein Austausch in einfacher Weise ermöglicht wird.

Bei einer weiteren bevorzugten Weiterbildung können das erste und das zweite Befestigungsteil jeweils eine Lochanordnung aufweisen, durch deren Löcher jeweils diesen zugeordnete Festlegemittel in eine Struktur des Unterkiefers bzw. des Craniums eingreifen können. Die jeweilige Lochanordnung kann sowohl hinsichtlich der Positionierung wie der Anzahl der Löcher an die jeweiligen örtlichen Gegebenheiten angepasst sein. Beispielsweise kann das erste Befestigungsteil des ersten Implantatteils mit sechs Löchern versehen sein, die gleichmäßig beabstandet an dem Befestigungssteil angeordnet sind, während die Lochanordnung des zweiten Befestigungsteils des zweiten Implantatteils mit lediglich vier Löchern versehen ist. Es sind aber jeweils auch andere Lochanordnungen denkbar. Durch die Löcher der Lochanordnung können etwa Schrauben als Festlegemittel greifen. Die Festlegemittel sind nicht auf Schrauben beschränkt.

Die obige Aufgabe wird ebenfalls gelöst durch ein Verfahren zur Herstellung einer implantierbaren Kiefergelenksprothese mit den Merkmalen des Anspruchs 12.

Durch die ineinandergreifende einteilige Verbindung von Metallkomponente und Kunststoffkomponenten wird eine stabile Gelenkfläche für die Gleitpaarung ausgebildet.

Bei einer bevorzugten Weiterbildung des erfindungsgemäßen Herstellungsverfahrens, die eine erhöhte Stabilität und Haltbarkeit der Gelenkfläche bietet, werden während des Verbindens der ersten und zweiten Verbindungsfläche die erste und zweite Komponente mit Temperatur sowie Druck und/oder Vakuum beaufschlagt.

Erfindungsgemäß werden die erste Komponente und die zweite Komponente beim Verbinden miteinander zu einem Schmelzverbund verschmolzen, so dass die Komponenten in einem Stoffschluss miteinander zusammengefügt werden.

Bei einer weiteren bevorzugten Weiterbildung des erfindungsgemäßen Herstellungsverfahrens wird der Schmelzverbund an der ersten und zweiten Verbindungsfläche ausgebildet, indem die erste Komponente in eine Schmelzform eingelegt und ein von der ersten Komponente eingefasstes Volumen im Bereich der Gelenkfläche vor der Beaufschlagung mit Temperatur, Druck und/oder Vakuum mit dem Kunststoff der zweiten Komponente in Pulverform zumindest teilweise aufgefüllt wird.

Dabei wird bei einer weiteren bevorzugten Weiterbildung des erfindungsgemäßen Herstellungsverfahrens die Schmelzform mehrteilig ausgebildet und umschließt die erste Komponente und die zweite Komponente beim Verbinden miteinander zu einem Schmelzverbund vollständig, so dass die Komponenten in geeigneter Weise einteilig verbunden werden und anschließend ohne Schwierigkeiten aus der Schmelzform entformt werden können.

Die obigen Ausgestaltungen und Weiterbildungen lassen sich, sofern sinnvoll, beliebig miteinander kombinieren. Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmalen der Erfindung.

Die Erfindung wird nachstehend anhand von Ausführungsbeispielen in den Figuren der Zeichnung näher erläutert. In teilweise schematisierter Darstellung zeigen hierbei:
- Fig. 1: eine perspektivische Seitenansicht einer ersten erfindungsgemäßen Ausführungsform der Kiefergelenksprothese mit einem ersten und zweiten Implantatteil im implantierten Zustand;
- Fig. 2a), b): perspektivische Seitenansichten der ersten Komponente und zweiten Komponente des zweiten Implantatteils der ersten Ausführungsform;
- Fig. 3a), b): perspektivische Seitenansichten der ersten Komponente und zweiten Komponente des zweiten Implantatteils einer zweiten Ausführungsform;
- Fig. 4: eine explosionsartige perspektivische Seitenansicht einer dritten Ausführungsform, wobei die erste Komponente des zweiten Implantatteils in eine Schmelzform zum Verbinden mit der zweiten Komponente eingelegt ist;
- Fig. 5a)-5d): perspektivische Seitenansichten der dritten Ausführungsform, welche das Verbinden der ersten Komponente mit der zweiten Komponente des zweiten Implantatteils in der Schmelzform in dessen zeitlichem Ablauf zeigen;
- Fig. 6: eine perspektivische Seitenansicht einer Rückseite von einem zweiten Implantatteil mit verbundenen Komponenten einer vierten Ausführungsform; und
- Fig. 7: eine perspektivische Seitenansicht einer Rückseite von einem zweiten Implantatteil mit verbundenen Komponenten einer fünften Ausführungsform.

In sämtlichen Figuren sind gleiche bzw. funktionsgleiche Elemente und Vorrichtungen - sofern nichts anderes angegeben ist - mit denselben Bezugszeichen versehen worden.

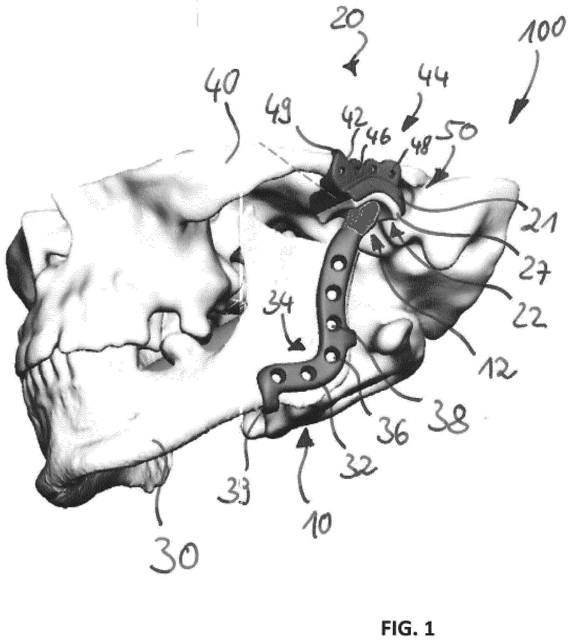

Fig. 1 zeigt eine perspektivische Seitenansicht einer ersten Ausführungsformen der erfindungsgemäßen Kiefergelenksprothese mit einem ersten und zweiten Implantatteil im implantierten Zustand.

In der Fig. 1 ist eine im Ganzen mit 100 bezeichnete implantierbare Kiefergelenksprothese mit einem ersten Implantatteil 10 und einem zweiten Implantatteil 20, die gemeinsam eine Gleitpaarung bilden, gezeigt. Das erste Implantatteil 10 ist dabei an dem dargestellten Unterkiefer 30 (Mandibula) befestigt und ist an seinem dem zweiten Implantatteil 20 zugewandten Ende mit einem Gelenkkopf 12 versehen. Das zweite Implantatteil 20 ist am Jochbein (Os zygomaticum) des Craniums 40 befestigt und mit einer dem Gelenkkopf 12 zugewandten Gelenkfläche 22 versehen, die für den Gelenkkopf 12 des ersten Implantatteils 10 ein Widerlager bildet.

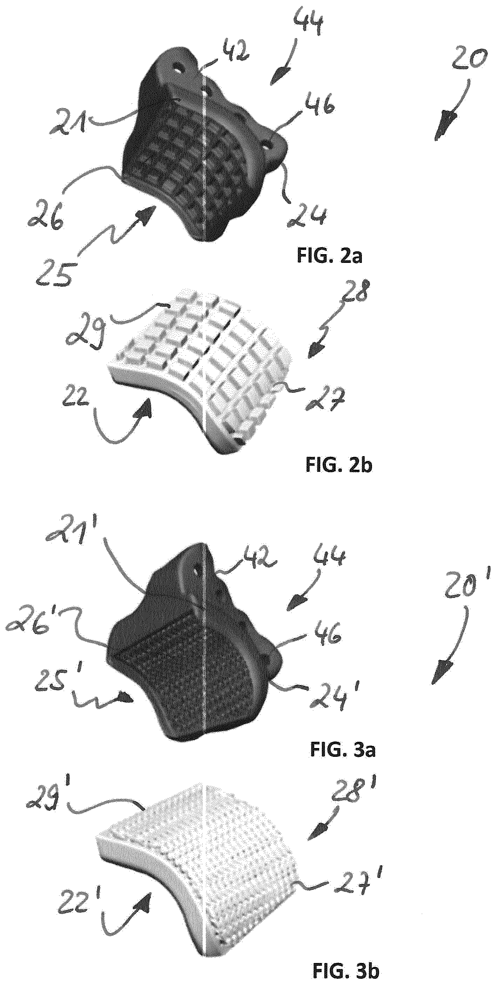

Dabei weist das zweite Implantatteil 20 eine aus einem metallischen Werkstoff gebildete erste Komponente 24 auf, die an dem Cranium 40 befestigt ist, während die Gelenkfläche 22 aus einer aus einem Kunststoff gebildeten zweiten Komponente 27 ausgebildet ist. Die erste Komponente 24 weist dabei eine erste Verbindungsfläche 25 auf, und die zweite Komponente 27 weist eine zweite Verbindungsfläche 28 auf (vgl. Fig. 2 und 3), die in der Fig. 1 deswegen nicht erkennbar sind, weil die Verbindungsflächen 25, 28 einander zugewandt und die erste Verbindungsfläche 25 und die zweite Verbindungsfläche 28 ineinandergreifend miteinander einteilig verbunden sind, so dass man in der Fig. 1 lediglich die an der den Verbindungsflächen 25, 28 abgewandten Seite ausgebildete, dem Betrachter zugewandte Gelenkfläche erkennt.

An dem zweiten Implantatteil 20 ist die erste Komponente 24 aus Titan und der Kunststoff der zweiten Komponente 27 aus einem thermoplastischem Kunststoff, z.B. PE-UHMW (ultrahochmolekulares Polyethylen), ausgebildet, während das erste Implantatteil 10 beispielsweise aus Titan ausgebildet ist. Die erste Komponente 24 und die zweite Komponente 27 des zweiten Implantatteils 20 bilden mit ihren ineinandergreifenden Verbindungsflächen 25, 28 einen Schmelzverbund 50.

An der ersten Komponente 24 des zweiten Implantatteils 20 ist an der Gelenkfläche 22 ein Randbereich 21 ausgebildet ist, der die Gelenkfläche 22 an der dem Betrachter zugewandten Seite und an der dem Betrachter abgewandten Seiten des zweiten Implantatteils 20 bereichsweise, umgreift.

Weiter zeigt die Fig. 1, dass die beiden Implantatteile 10, 20 jeweils mittels eines jeweiligen Befestigungsteils 32, 42, also mittels eines ersten bzw. zweiten Befestigungsteils 32, 42, an dem Unterkiefer 30 bzw. dem Cranium 40 lösbar befestigt sind. Dabei weist das erste Befestigungsteil 32 eine erste Lochanordnung 34 und das zweite Befestigungsteil 42 eine zweite Lochanordnung 44 auf, durch deren Löcher 36, 46 Schrauben als Festlegemittel 38, 48 die jeweilige Struktur des Unterkiefers 30 bzw. des Craniums 40 greifen.

Das erste Implantatteil 10 ist in Art eines flachen Bügels ausgebildet, dessen Krümmung derjenigen des aufsteigenden Astes der Mandibula 30 folgt und ist an dieser mit sechs Schrauben 38 festgelegt. Diese Schrauben 38 greifen durch gleichmäßig beabstandete Löcher 36 der ersten Lochanordnung 34. An dem dem Gelenkkopf 12 abgewandten Ende sowie etwa in dessen Mitte ist an dem ersten Befestigungsteil 32 jeweils ein Steg 39 angeordnet, der quer von dem Befestigungsteil vorspringt und den Knochen der Mandibula 30 umgreift.

Das zweite Implantatteil 20 weist in seinem der Gelenkfläche 22 abgewandten Bereich das zweite Befestigungsteil 42 auf, das mittig von der ersten Verbindungsfläche 25 der ersten Komponente in der zweiten Verbindungsfläche 28 abgewandter Richtung erstreckt und eine zweite Anordnung 44 gleichmäßig beabstandeter Löcher 46 aufweist. Schrauben 48 als Festlegemittel halten das zweite Implantatteil 20 an dem Jochbein des Craniums 40. Überdies ist an dem frontalen Ende des zweiten Befestigungsteils 42 ein das Jochbein des Craniums 40 umgreifender Steg 49 angeordnet.

Fig. 2a), b) zeigen perspektivische Seitenansichten der ersten Komponente und zweiten Komponente des zweiten Implantatteils 20 der ersten Ausführungsform, wie bereits im Zusammenhang mit Fig. 1 teilweise erläutert.

Gemäß Fig. 2a) sind die Ausnehmungen 26 der ersten Verbindungsfläche 25 gleichmäßig verteilt, wobei gemäß Fig. 2b) die quaderförmigen Vorsprünge 29 der zweiten Verbindungsfläche 28 diese im verbundenen Zustand aufnehmen.

Fig. 3a), b) zeigen perspektivische Seitenansichten der ersten Komponente und zweiten Komponente des zweiten Implantatteils 20' einer zweiten Ausführungsform.

Die zweite Ausführungsform unterscheidet sich lediglich hinsichtlich der Ausbildung der Ausnehmungen 26' an der ersten Komponente 24' sowie den zugeordneten Vorsprüngen 29' an der zweiten Komponente 27'. Dabei zeigt Fig. 3a) die erste Komponente 24' mit der ersten Verbindungsfläche 25' und dem Randbereich 21', die dem Betrachter zugewandt ist, so dass die Ausnehmungen 26' deutlich zu erkennen sind, in welche bei Verbindung mit der zweiten Verbindungsfläche 28' an dieser angeordnete Vorsprünge 29' gemäß Fig. 3b) eingreifen.

Die Vorsprünge 29' der zweiten Verbindungsfläche 28' gemäß der Fig. 3b) sind als erhabene Polygonzüge ausgebildet, welche sich untereinander in der Formgebung unterscheiden und insoweit inhomogen sind.

Die Verbindungsflächen 25, 25', 28, 28' der ersten Komponente 24, 24' und der zweiten Komponente 27, 27' sind als zueinander kongruente Flächen ausgebildet, die einander überdecken. Außerdem bildet die Verbindungsfläche 25, 25' der ersten Komponente 24, 24' im Querschnitt eine konkave Form und weist dabei eine Krümmung mit sich änderndem Krümmungsradius auf. Außerdem weist das zweite Implantatteil 20, 20' an der Gelenkfläche 22, 22' einen Randbereich 21, 21 ` aufweist, der die Gelenkfläche 22, 22' bereichsweise umgreift.

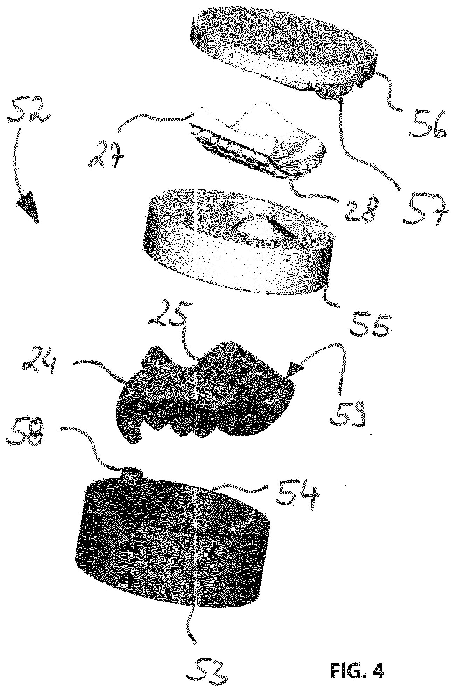

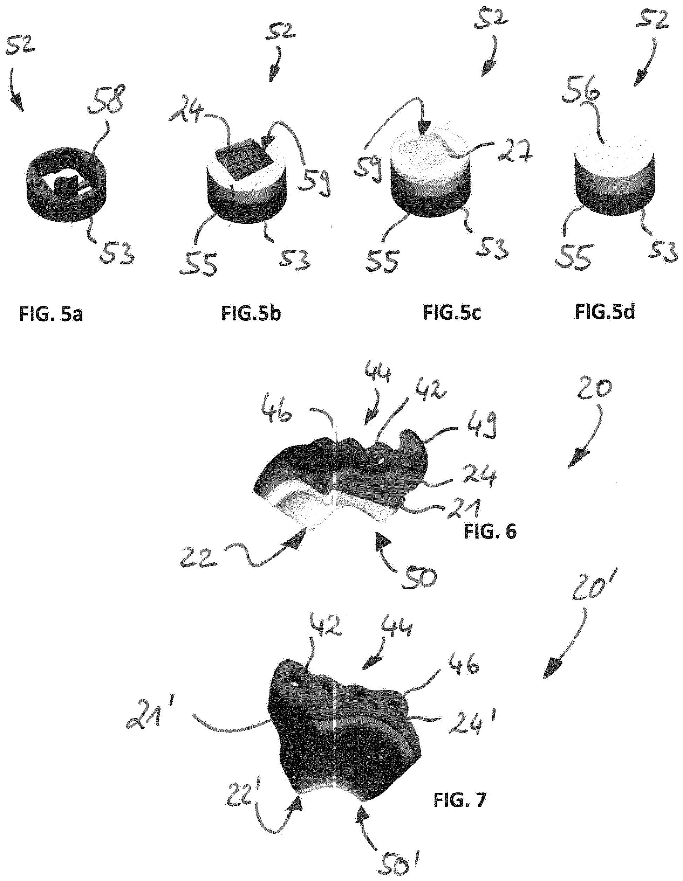

Fig. 4 ist eine explosionsartige perspektivische Seitenansicht einer dritten Ausführungsform, wobei die erste Komponente des zweiten Implantatteils in eine Schmelzform zum Verbinden mit der zweiten Komponente eingelegt ist, und die Fig. 5a-5d sind perspektivische Seitenansichten der dritten Ausführungsform, welche das Verbinden der ersten Komponente mit der zweiten Komponente des zweiten Implantatteils in der Schmelzform in dessen zeitlichem Ablauf zeigen..

In den Fig. 4 und 5 ist eine Ausführungsform des Herstellverfahrens für das zweite Implantatteil 20, 20' der Kiefergelenkprothese 100 schematisch dargestellt und wird mit Bezug auf das zweite Implantatteil 20 erläutert. Gemäß Fig. 4 ist die erste Komponente 24 des zweiten Implantatteils 20 in eine Schmelzform 52 eingelegt ist, damit sie mit der zweiten Komponente 27 verbunden werden kann. Die Schmelzform 52 ist dabei in einer Zylinderform ausgebildet, die mit einem Unterteil 53, einem Mittelteil 55 und einem Deckelteil 56 versehen ist. An dem Unterteil ist ein Stützelement 54 ausgebildet, welches die erste Komponente 24 des zweiten Implantatteils 20 beim Einlegen abstützt, während das Mittelteil 55 die erste Komponente umgreift. Zum Verbinden der Komponenten 24, 27 wird das Volumen 59, das durch die erste Komponente 24 an deren erster Verbindungsfläche 25 eingefasst ist, mit dem Kunststoff der zweiten Komponente 27 in Pulverform aufgefüllt (in Fig. 4 nicht gezeigt), um die gezeigte zweite Komponente 27 auszubilden. Zum Verbinden unter Beaufschlagung mit Temperatur Druck und Vakuum wird die Schmelzform 52 mit einem stempelartigen Deckelteil 56 verschlossen, dessen der ersten Komponente zugewandte Formseite mit einem gewölbten Bereich 57 in Richtung der sich in der Schmelzform 52 befindlichen ersten Komponente 24 gewölbt ist.

Die Fig. 5a) bis 5d) zeigen den bereits zuvor skizzierten zeitlichen Ablauf des Verschmelzens der Komponenten, indem in Fig. 5a) das Unterteil 53 der im Wesentlichen zylindrischen Schmelzform 52 dargestellt ist, die in ihrem Innern mit dem Stützteil 54 zur Abstützung der in sie einzulegenden ersten Komponente 24 des zweiten Implantatteils 20 versehen ist. Weiter erkennt man in der Fig. 5a) zwei einander an dem dem nicht dargestellten Mitteilteil 55 zugewandten stirnseitigen Rand angeordneten Haltevorsprünge 58. Der erwähnte Mittelteil 55 umgreift in der Fig. 5b) die in die Schmelzform 52 eingelegte erste Komponente, durch deren Wölbung in Richtung des Unterteils 53 das Volumen 59 ausgebildet ist. Dieses ist in der Fig. 5c) durch die zweite Komponente 27 in Pulverform teilweise aufgefüllt, die Schmelzform wird anschließend in der Fig. 5d) mit Temperatur, Druck und Vakuum beaufschlagt, so dass nach Verschließen der Schmelzform 52 mittels des Deckels 56 mit dem in Richtung des Unterteils 53 vorgewölbten Deckelbereich 57 der Schmelzverbund 50 an dem zweiten Implantatteil 20 ausgebildet werden kann.

Fig. 6 zeigt eine perspektivische Seitenansicht einer Rückseite von einem zweiten Implantatteil mit verbundenen Komponenten einer vierten Ausführungsform, und Fig. 7 zeigt eine perspektivische Seitenansicht einer Rückseite von einem zweiten Implantatteil mit verbundenen Komponenten einer fünften Ausführungsform.

Es ist erkennbar, dass sich trotz unterschiedlich ausgebildeter erster und zweiter Verbindungsflächen 25, 25', 28, 28' an den beiden zweiten Implantatteilen 20, 20' diese nach außen hin identisch gestaltet sind, insbesondere eine Gelenkfläche 22, 22' mit gleichwirkender Funktionalität zur Verfügung stellen.

Obwohl die vorliegende Erfindung anhand bevorzugter Ausführungsbeispiele vorstehend beschrieben wurde, ist sie darauf nicht beschränkt, sondern auf vielfältige Art und Weise modifizierbar. Insbesondere lässt sich die Erfindung in mannigfaltiger Weise verändern oder modifizieren, ohne vom Kern der Erfindung abzuweichen.

## Patentansprüche

1. Implantierbare Kiefergelenksprothese (100) mit:
einem ersten Implantatteil (10) mit einem künstlichen Gelenkkopf (12), welches an einem Unterkiefer (30) befestigbar ist;
einem zweiten Implantatteil (20, 20') mit einer Gelenkfläche (22, 22'), welches an einem Cranium (40) befestigbar ist;
wobei die Gelenkfläche (22, 22') ein Widerlager für den künstlichen Gelenkkopf (12) bildet;
wobei das zweite Implantatteil (20, 20') eine aus einem metallischen Werkstoff gebildete erste Komponente (24, 24') aufweist, welche an dem Cranium (40) befestigbar ist, und eine aus einem Kunststoff gebildete zweite Komponente (27, 27') aufweist, welche die Gelenkfläche (22, 22') ausbildet;
wobei die erste Komponente (24, 24') eine erste Verbindungsfläche (25, 25') aufweist und die zweite Komponente (27, 27') eine zweite Verbindungsfläche (28, 28') aufweist, und die erste Verbindungsfläche (25, 25') und die zweite Verbindungsfläche (28, 28') ineinandergreifend miteinander einteilig verbunden sind;
**dadurch gekennzeichnet, dass**
die erste Komponente (24, 24') und die zweite Komponente (27, 27') an der ersten Verbindungsfläche (25, 25') und der zweiten Verbindungsfläche (28, 28') einen Schmelzverbund (50, 50') ausbilden.

2. Kiefergelenksprothese nach Anspruch 1, wobei die erste Verbindungsfläche (25, 25') mit Ausnehmungen (26, 26') versehen ist, in welche an der zweiten Verbindungsfläche (28, 28') angeordnete Vorsprünge (29, 29') eingreifen.

3. Kiefergelenksprothese nach Anspruch 2, wobei die Ausnehmungen (26, 26') in der ersten Verbindungsfläche (25, 25') der ersten Komponente (24, 24') und die entsprechenden Vorsprünge (29, 29') der zweiten Komponente (27, 27') homogen oder inhomogen ausgebildet sind.

4. Kiefergelenksprothese nach Anspruch 2 oder 3, wobei die Größe der Ausnehmungen (26, 26') an der ersten Verbindungsfläche (25, 25') der ersten Komponente (24, 24') zwischen 1 µm und 2000 µm beträgt.

5. Kiefergelenksprothese nach einem der vorhergehenden Ansprüche, wobei die erste Verbindungsfläche (25, 25') und die zweite Verbindungsfläche (28, 28') als zueinander kongruente Flächen ausgebildet sind.

6. Kiefergelenksprothese nach einem der vorhergehenden Ansprüche, wobei die erste Verbindungsfläche (25, 25') konkav ausgebildet ist.

7. Kiefergelenksprothese nach einem der vorhergehenden Ansprüche, wobei die erste Komponente (24, 24') aus Titan oder einer Titanlegierung und der Kunststoff der zweiten Komponente (27, 27') aus einem thermoplastischen Kunststoff, insbesondere aus PE-UHMW als thermoplastischem Kunststoff, ausgebildet ist.

8. Kiefergelenksprothese nach einem der vorhergehenden Ansprüche, wobei das erste Implantatteil (10) aus einer Keramik, einem PEEK-Werkstoff, einem metallischen Werkstoff oder einer Kombination dieser Werkstoffe ausgebildet ist.

9. Kiefergelenksprothese nach einem der vorhergehenden Ansprüche, wobei die erste Komponente (24, 24') einen Randbereich (21, 21') aufweist, der die zweite Komponente (27, 27') zumindest bereichsweise umgreift.

10. Kiefergelenksprothese nach einem der vorhergehenden Ansprüche, wobei das erste und das zweite Implantatteil (10, 20, 20') mittels eines ersten bzw. zweiten Befestigungsteils (32, 42, 42') an dem Unterkiefer (30) bzw. dem Cranium (40) lösbar befestigbar sind.

11. Kiefergelenksprothese nach Anspruch 10, wobei das erste Befestigungsteil (32) eine erste Lochanordnung (34) und das zweite Befestigungsteil (42, 42') eine zweite Lochanordnung (44, 44') aufweist.

12. Verfahren zur Herstellung einer implantierbaren Kiefergelenksprothese (10), zumindest mit den folgenden Schritten:
Bereitstellen eines ersten Implantatteils (10) mit einem künstlichen Gelenkkopf (12), welches an einem Unterkiefer (30) befestigbar ist;
Bereitstellen eines zweiten Implantatteils (20, 20') mit einer Gelenkfläche (22, 22'), welches an einem Cranium (40) befestigbar ist;
wobei die Gelenkfläche (22, 22') ein Widerlager für den künstlichen Gelenkkopf (12) bildet;
wobei das zweite Implantatteil (20, 20') eine erste, aus einem metallischen Werkstoff gebildete erste Komponente (24, 24') aufweist, welche an dem Cranium (40) befestigbar ist, und eine zweite, aus einem Kunststoff gebildete Komponente (27, 27') aufweist, welche die Gelenkfläche (22, 22') ausbildet;
ineinandergreifendes einteiliges Verbinden einer ersten Verbindungsfläche (25, 25') der ersten Komponente (24, 24') mit einer zweiten Verbindungsfläche (28, 28') der zweiten Komponente (27, 27');
**dadurch gekennzeichnet, dass**
die erste Komponente (24, 24') und die zweite Komponente (27, 27') bei Verbinden an der ersten Verbindungsfläche (25, 25') und der zweiten Verbindungsfläche (28, 28') zu einem Schmelzverbund (50, 50') verschmolzen werden.

13. Verfahren nach Anspruch 12, wobei während des Verbindens der ersten und zweiten Verbindungsfläche (25, 25', 28, 28') die erste Komponente (24, 24') und die zweite Komponente (27, 27') mit Temperatur sowie Druck und/oder Vakuum beaufschlagt werden.

14. Verfahren nach Anspruch 12 oder 13, wobei der Schmelzverbund (50, 50') an der ersten und zweiten Verbindungsfläche (25, 25', 28, 28') ausgebildet wird, indem die erste Komponente (24, 24') in eine Schmelzform (52) eingelegt und ein von der ersten Komponente (24, 24') eingefasstes Volumen (54) im Bereich der Gelenkfläche (22, 22') vor der Beaufschlagung mit Temperatur, Druck und Vakuum mit dem Kunststoff der zweiten Komponente (27, 27') in Pulverform zumindest teilweise aufgefüllt wird.

15. Verfahren nach Anspruch 14, wobei die Schmelzform (52) mehrteilig ausgebildet ist und die erste Komponente (24, 24') und die zweite Komponente (27, 27') bei deren Verbinden miteinander zu einem Schmelzverbund (50, 50') vollständig umschließt.

## Claims

1. Implantable temporomandibular joint prosthesis (100), comprising:
a first implant part (10) having an artificial condyle (12) which is attachable to a lower jaw (30);
a second implant part (20, 20') having an articular surface (22, 22') which is attachable to a cranium (40);
the articular surface (22, 22') forming an abutment for the artificial condyle (12);
the second implant part (20, 20') having a first component (24, 24') formed from a metal material and attachable to the cranium (40) and having a second component (27, 27') formed from a plastics material and forming the articular surface (22, 22');
the first component (24, 24') having a first connection surface (25, 25') and the second component (27, 27') having a second connection surface (28, 28'), and the first connection surface (25, 25') and the second connection surface (28, 28') being integrally connected so as to engage in one another;
**characterised in that**
the first component (24, 24') and the second component (27, 27') form a fused composite (50, 50') at the first connection surface (25, 25') and the second connection surface (28, 28').

2. Temporomandibular joint prosthesis according to claim 1, wherein the first connection surface (25, 25') is provided with recesses (26, 26'), in which projections (29, 29') arranged on the second connection (28, 28') surface engage.

3. Temporomandibular joint prosthesis according to claim 2, wherein the recesses (26, 26') in the first connection surface (25, 25') of the first component (24, 24') and the corresponding projections (29, 29') of the second component (27, 27') are configured homogeneous or inhomogeneous.

4. Temporomandibular joint prosthesis according to either claim 2 or claim 3, wherein the size of the recesses (26, 26') on the first connection surface (25, 25') of the first component (24, 24') is between 1 µm and 2000 µm.

5. Temporomandibular joint prosthesis according to any of the preceding claims, wherein the first connection surface (25, 25') and the second connection surface (28, 28') are configured as mutually congruent surfaces.

6. Temporomandibular joint prosthesis according to any of the preceding claims, wherein the first connection surface (25, 25') is configured concave.

7. Temporomandibular joint prosthesis according to any of the preceding claims, wherein the first component (24, 24') is made of titanium or a titanium alloy, and the plastics material of the second component is made of a thermoplastic, in particular of PE-UHMW as a thermoplastic.

8. Temporomandibular joint prosthesis according to any of the preceding claims, wherein the first implant part (10) is made of a ceramic, a PEEK material, a metal material or a combination of these materials.

9. Temporomandibular joint prosthesis according to any of the preceding claims, wherein the first component (24, 24') has an edge region (21, 21') which engages around the second component (27, 27') at least in regions.

10. Temporomandibular joint prosthesis according to any of the preceding claims, wherein the first and the second implant part (10, 20, 20') are detachably attachable to the lower jaw (30) and the cranium (40) respectively by means of a first and a second attachment part (32, 42, 42') respectively.

11. Temporomandibular joint prosthesis according to claim 10, wherein the first attachment part (32) has a first hole arrangement (34) and the second attachment part (42, 42') has a second hole arrangement (44, 44').

12. Method for manufacturing an implantable temporomandibular joint prosthesis (10), at least comprising the following steps:
providing a first implant part (10) having an artificial condyle (12) which is attachable to a lower jaw (30);
providing a second implant part (20, 20') having an articular surface (22, 22') which is attachable to a cranium (40);
the articular surface (22, 22') forming an abutment for the artificial condyle (12);
the second implant part (20, 20') having a first first component (24, 24'), which is formed from a metal material and is attachable to the cranium (40), and having a second component (27, 27'), which is formed from a plastics material and forms the articular surface (22, 22');
integrally connecting a first connection surface (25, 25') of the first component (24, 24') to a second connection surface (28, 28') of the second component (27, 27') in such a way that they engage with one another;
**characterised in that**
the first component (24, 24') and the second component (27, 27') are fused together during connection at the first connection surface (25, 25') and the second connection surface (28, 28') so as to form a fused composite (50, 50').

13. Method according to claim 12, wherein heat as well as pressure and/or vacuum are applied to the first component (24, 24') and the second component (27, 27') during connection of the first and the second connection surface (25, 25', 28, 28').

14. Method according to either claim 12 or claim 13, wherein the fused composite (50, 50') is formed at the first and the second connection surface (25, 25', 28, 28') by inserting the first component (24, 24') into a fusion mould (52) and at least partially filling a volume (54), encompassed by the first component (24, 24'), in the region of the articular surface (22, 22') with the plastics material of the second component (27, 27') in powder form before the application of heat, pressure and vacuum.

15. Method according to claim 14, wherein the fusion mould (52) is configured in multiple pieces and completely encloses the first component (24, 24') and the second component (27, 27') during the interconnection thereof to form a fused composite (50, 50').

## Revendications

1. Prothèse d'articulation temporo-mandibulaire (100) implantable comprenant :
une première partie d'implant (10) comportant un condyle artificiel (12) qui peut être fixée à une mâchoire inférieure (30) ;
une seconde partie d'implant (20, 20') comportant une surface d'articulation (22, 22') qui peut être fixée à un crâne (40) ;
dans laquelle la surface d'articulation (22, 22') forme une butée pour le condyle artificiel (12) ;
dans laquelle la seconde partie d'implant (20, 20') présente un premier composant (24, 24') qui est formé d'un matériau métallique et peut être fixé au crâne (40), et présente un second composant (27, 27') qui est formé d'une matière plastique et forme la surface d'articulation (22, 22') ;
dans laquelle le premier composant (24, 24') présente une première surface de liaison (25, 25') et le second composant (27, 27') présente une seconde surface de liaison (28, 28'), et la première surface de liaison (25, 25') et la seconde surface de liaison (28, 28') sont reliées l'une à l'autre en une seule pièce en s'imbriquant l'une dans l'autre ;
**caractérisée en ce que**
le premier composant (24, 24') et le second composant (27, 27') forment, sur la première surface de liaison (25, 25') et la seconde surface de liaison (28, 28'), un composite assemblé par fusion (50, 50').

2. Prothèse d'articulation temporo-mandibulaire selon la revendication 1, dans laquelle la première surface de liaison (25, 25') est pourvue d'évidements (26, 26') dans lesquels s'imbriquent des saillies (29, 29') disposées sur la seconde surface de liaison (28, 28').

3. Prothèse d'articulation temporo-mandibulaire selon la revendication 2, dans laquelle les évidements (26, 26') dans la première surface de liaison (25, 25') du premier composant (24, 24') et les saillies (29, 29') correspondantes du second composant (27, 27') sont conçus de manière homogène ou non homogène.

4. Prothèse d'articulation temporo-mandibulaire selon la revendication 2 ou 3, dans laquelle la taille des évidements (26, 26') sur la première surface de liaison (25, 25') du premier composant (24, 24') est comprise entre 1 µm et 2000 µm.

5. Prothèse d'articulation temporo-mandibulaire selon l'une des revendications précédentes, dans laquelle la première surface de liaison (25, 25') et la seconde surface de liaison (28, 28') sont conçues sous la forme de surfaces congruentes.

6. Prothèse d'articulation temporo-mandibulaire selon l'une des revendications précédentes, dans laquelle la première surface de liaison (25, 25') est conçue concave.

7. Prothèse d'articulation temporo-mandibulaire selon l'une des revendications précédentes, dans laquelle le premier composant (24, 24') est conçu en titane ou en un alliage de titane et la matière plastique du second composant (27, 27') en une matière thermoplastique, en particulier en PE-UHMW en guise de matière thermoplastique.

8. Prothèse d'articulation temporo-mandibulaire selon l'une des revendications précédentes, dans laquelle la première partie d'implant (10) est conçue à partir d'une céramique, d'un matériau PEEK, d'un matériau métallique ou d'une combinaison de ces matériaux.

9. Prothèse d'articulation temporo-mandibulaire selon l'une des revendications précédentes, dans laquelle le premier composant (24, 24') présente une zone de bord (21, 21') qui entoure, au moins par endroits, le second composant (27, 27').

10. Prothèse d'articulation temporo-mandibulaire selon l'une des revendications précédentes, dans laquelle la première et la seconde partie d'implant (10, 20, 20') peuvent être fixées de manière détachable respectivement à la mâchoire inférieure (30) et au crâne (40) au moyen respectivement d'une première et d'une seconde partie de fixation (32, 42, 42').

11. Prothèse d'articulation temporo-mandibulaire selon la revendication 10, dans laquelle la première partie de fixation (32) présente un premier agencement de trous (34) et la seconde partie de fixation (42, 42') présente un second agencement de trous (44, 44').

12. Procédé de fabrication d'une prothèse d'articulation temporo-mandibulaire (10) implantable, comprenant au moins les étapes suivantes :
mise à disposition d'une première partie d'implant (10) comportant un condyle artificiel (12) qui peut être fixée à une mâchoire inférieure (30) ;
mise à disposition d'une seconde partie d'implant (20, 20') comportant une surface d'articulation (22, 22') qui peut être fixée à un crâne (40) ;
dans lequel la surface d'articulation (22, 22') forme une butée pour le condyle artificiel (12) ;
dans lequel la seconde partie d'implant (20, 20') présente un premier premier composant (24, 24') qui est formé d'un matériau métallique et peut être fixé au crâne (40), et présente un second composant (27, 27') qui est formé d'une matière plastique et forme la surface d'articulation (22, 22') ;
liaison en une seule pièce, avec imbrication l'une dans l'autre, d'une première surface de liaison (25, 25') du premier composant (24, 24') et d'une seconde surface de liaison (28, 28') du second composant (27, 27') ;
**caractérisé en ce que**
lors de la liaison sur la première surface de liaison (25, 25') et la seconde surface de liaison (28, 28'), le premier composant (24, 24') et le second composant (27, 27') sont fondus pour former un composite assemblé par fusion (50, 50').

13. Procédé selon la revendication 12, dans lequel, pendant la liaison de la première et la seconde surface de liaison (25, 25', 28, 28'), le premier composant (24, 24') et le second composant (27, 27') sont soumis à l'action de la chaleur ainsi que de la pression et/ou du vide.

14. Procédé selon la revendication 12 ou 13, dans lequel le composite assemblé par fusion (50, 50') est formé sur la première et la seconde surface de liaison (25, 25', 28, 28') en insérant le premier composant (24, 24') dans un moule de fusion (52) et en remplissant, au moins en partie, un volume (54) bordé par le premier composant (24, 24') dans la zone de la surface d'articulation (22, 22') avec la matière plastique du second composant (27, 27') sous forme de poudre avant la soumission à l'action de la chaleur, de la pression et du vide.

15. Procédé selon la revendication 14, dans lequel le moule de fusion (52) est conçu en plusieurs parties et entoure entièrement le premier composant (24, 24') et le second composant (27, 27') lors de leur liaison l'un à l'autre pour former un composite assemblé par fusion (50, 50').
